(19) Europäisches Patentamt
European Patent Office
Office européen des brevets

(11) EP 4 093 727 B1

(12) # EUROPEAN PATENT SPECIFICATION

(45) Date of publication and mention of the grant of the patent:
**31.07.2024 Bulletin 2024/31**

(21) Application number: **21701697.1**

(22) Date of filing: **20.01.2021**

(51) International Patent Classification (IPC):
***C07C 251/20*** (2006.01) ***A61K 8/41*** (2006.01)
***A61P 17/16*** (2006.01)

(52) Cooperative Patent Classification (CPC):
**C07C 251/20; A61K 8/41; A61P 17/16; A61Q 17/04; A61Q 19/00;** A61K 2800/10; A61K 2800/522; C07C 2601/16

(86) International application number:
**PCT/EP2021/051112**

(87) International publication number:
**WO 2021/148426 (29.07.2021 Gazette 2021/30)**

(54) **ANALOGUES OF MYCOSPORINE-LIKE AMINO ACID AND USE THEREOF AS SUNSCREENS**

ANALOGA VON MYCOSPORIN-ÄHNLICHEN AMINOSÄUREN UND DEREN VERWENDUNG ALS SONNENSCHUTZ

ANALOGUES D'ACIDES AMINÉS DE TYPE MYCOSPORINE ET LEUR UTILISATION COMME ECRAN SOLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2020 EP 20382031**

(43) Date of publication of application:
**30.11.2022 Bulletin 2022/48**

(73) Proprietor: **BASF SE**
**67056 Ludwigshafen am Rhein (DE)**

(72) Inventors:
- **SAMPEDRO, Diego**
  **26006 Logrono (ES)**
- **LOSANTOS, Raul**
  **26006 Logrono (ES)**
- **FUNES-ARDOIZ, Ignacio**
  **26006 Logrono (ES)**
- **EHLIS, Thomas**
  **4133 Schweizerhalle (Muttenz) (CH)**
- **GIESINGER, Jochen**
  **79639 Grenzach-Wyhlen (DE)**
- **GRUMELARD, Julie**
  **79639 Grenzach-Wyhlen (DE)**

(74) Representative: **BASF IP Association**
**BASF SE**
**GBI-C006**
**67056 Ludwigshafen (DE)**

(56) References cited:
**ES-A1- 2 550 374**

- **MARIA ORFANOUDAKI ET AL: "Chemical profiling of mycosporine-like amino acids in twenty-three red algal species", JOURNAL OF PHYCOLOGY., vol. 55, no. 2, 1 April 2019 (2019-04-01), US, pages 393 - 403, XP055714863, ISSN: 0022-3646, DOI: 10.1111/jpy.12827**
- **RAJESH P. RASTOGI ET AL: "Characterization of UV-screening compounds, mycosporine-like amino acids, and scytonemin in the cyanobacterium Lyngbya sp. CU2555", FEMS MICROBIOLOGY ECOLOGY., vol. 87, no. 1, 15 October 2013 (2013-10-15), NL, pages 244 - 256, XP055381723, ISSN: 0168-6496, DOI: 10.1111/1574-6941.12220**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 093 727 B1

## Description

The present invention relates to the field of protection against ultraviolet (UV) radiation. In particular, the present invention relates to compounds of formula (I)

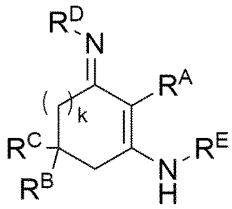

(I)

wherein $R^A$, $R^B$, $R^C$, $R^D$, and $R^E$ are as defined herein. Further, the present invention is concerned with compositions comprising at least one compound of formula (I). The present invention also relates to compounds of formula (I) and compositions comprising at least one compound of formula (I) for use in a sunscreen. Yet further, the present invention is concerned with compounds of formula (I) in body-care products and in household cleaning and treating agents as a light stabilizer.

UV radiation causes harmful effects on the human skin. Beside the acute effect of sunburn of the skin, UV radiation is also known to increase the risk of skin cancer. Furthermore, long time exposure to UV-A and UV-B light can cause phototoxic and photo allergenic reactions on the skin and can accelerate skin aging.

To protect the human skin from UV radiation, various sun protecting UV filters (also referred to as UV absorbers) exist including UV-A filter, UV-B filter and broadband filters. These filters are added to sunscreen or cosmetic compositions. The UV filters are either organic or inorganic, particulate or non-particulate compounds, of which all have a high absorption efficacy in the UV-light range. In general, UV light can be divided into UV-A radiation (320 - 400 nm) and UV-B radiation (280 - 320 nm). Depending on the position of the absorption maxima, UV filters are divided into UV-A and UV-B filters. In case an UV filter absorbs both, UV-A and UV-B light, it is referred to as a broadband absorber.

Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

However, the UV filters known in the prior art, which are used in e.g. sunscreen or cosmetic compositions have certain disadvantages. In particular, it is referred to the disadvantage that the vast majority of cosmetic registered organic UV filters are suitable for the application in the oil phase of a sunscreen emulsion, as they are miscible or soluble in oils. The solubility of each UV filter is thereby dependent of the oils used. Hence, the formulator of sunscreens is limited by the choice of certain oils and thus also limited by choosing exemplarily emollients to improve the sensory. This may negatively affect the compliance of consumer.

Accordingly, there is a need for effective, water soluble UV filters.

Mycosporine-like amino acids (MAAs) have been associated with the potential to protect organisms against UV radiation. MAAs consist of a family of compounds with a cyclohexenone or cyclohexenimine basic structure. Analogues of MAA are known to be suitable UV filters in terms of absorption and photostability (ES2550374B1 and Losantos et al., Angew. Chem. 2017, 129, 1-5), which are however nearly insoluble in water.

Hence, there is an ongoing need for water soluble novel compounds that sufficiently protect the skin from UV radiation. In this connection, it has been another object of the present invention to provide efficient UV protective compositions, which are suitable for improving the photostability of body-care products as well as of household cleaning and treating agents. In particular, it had been an object of the present invention to provide compositions, which are suitable for improving the photostability of sunscreen compositions.

It has surprisingly been found that at least one of these objects can be achieved by the composition according to the present invention.

In particular, the inventors of the present invention have found compounds that effectively absorb light in the UV range relevant for UV protection. These compounds are analogues of mycosporine-like amino acids (MAAs).

In a first aspect, the present invention therefore relates to a compound of formula (I)

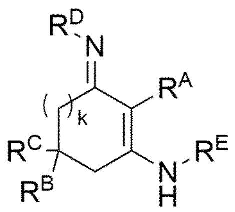

(I)

or a salt, stereoisomer, or tautomer thereof,
wherein

- $R^A$ is H, $C_1$-$C_6$-alkyl, $C_1$-$C_6$-haloalkyl, $C_1$-$C_6$-haloalkoxy, $C_2$-$C_6$-alkylene, (O-C(=O))-($C_1$-$C_4$-alkyl), (O-C(=O))-aryl, or S-($C_1$-$C_4$-alkyl);
- $R^B$ is H, halogen, hydroxy, hydroxymethyl, $C_1$-$C_6$-alkyl, $C_2$-$C_6$-alkylene, or $C_1$-$C_6$-alkoxy;
- $R^C$ is H, halogen, hydroxy, hydroxymethyl, $C_1$-$C_6$-alkyl, $C_2$-$C_6$-alkylene, or $C_1$-$C_6$-alkoxy;
- $R^D$ is $(CH_2O)_n$-$R^M$, $(CH_2CH_2O)_n$-$R^M$, or $(CH_2CH_2CH_2O)_n$-$R^M$;
- $R^E$ is $(CH_2O)_m$-$R^N$, $(CH_2CH_2O)_m$-$R^N$, or $(CH_2CH_2CH_2O)_m$-$R^N$;
- $R^M$ is H, $C_1$-$C_4$-alkyl, or saccharide;
- $R^N$ is H, $C_1$-$C_4$-alkyl, or saccharide;
- n is an integer from 1 to 50;
- m is an integer from 1 to 50; and
- k is 0, 1, or 2.

In the following, preferred embodiments of the substituents in the above formula (I) are described in further detail. It is to be understood that each preferred embodiment is relevant on its own as well as in combination with other preferred embodiments. Furthermore, it is to be understood that the preferences in each case also apply to the salts, stereoisomer, or tautomer of the compounds of the invention.

In a preferred embodiment A1 of the first aspect, the compound of formula (I) has the form of a salt according to formula (Ia)

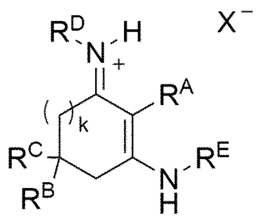

(Ia)

wherein
$X^-$ is halide, formate, acetate, succinate, oxalate, maleate, fumarate, malate, tartrate, citrate, trifluoroacetate, triflate, tosylate, or mesylate.

In a preferred embodiment A2 of the first aspect, k is 1.

In a preferred embodiment A3 of the first aspect, $R^A$ is methyl, ethyl, or thiomethyl.

In a preferred embodiment A4 of the first aspect, $R^B$ is H, halogen, hydroxyl, or hydroxymethyl and $R^C$ is H, halogen, hydroxyl, or hydroxymethyl.

In a preferred embodiment A5 of the first aspect, $R^D$ is $(CH_2CH_2O)_n$-$R^M$; $R^M$ is H or methyl; and n is an integer from 1 to 10.

In a preferred embodiment A6 of the first aspect, $R^E$ is $(CH_2CH_2O)_m$-$R^N$; $R^N$ is H or methyl; and m is an integer from 1 to 10.

In a preferred embodiment A7 of the first aspect, $R^D$ is $(CH_2CH_2O)_n$-$R^M$; $R^E$ is $(CH_2CH_2O)_m$-$R^N$; $R^M$ is H; $R^N$ is H; n is 1 or 2; and m is 1 or 2.

In a preferred embodiment A8 of the first aspect, $R^A$ is methyl.

In a preferred embodiment A9 of the first aspect, $R^B$ and $R^C$ are equal, preferably H.

In a preferred embodiment A10 of the first aspect, $X^-$ is chloride, tosylate, or mesylate.

In a preferred embodiment A11 of the first aspect, the compound according to formula (I) is selected from the group consisting of (E)-2-(2-hydroxyethoxy)-N-(3-((2-(2-hydroxyethoxy)ethyl)amino)-2-methylcyclohex-2-en-1 -ylidene)ethan-1 -aminium chloride and (E)-2-hydroxy-N-(3-((2-hydroxyethyl)amino)-2-methylcyclohex-2-en-1-ylidene)ethan-1-aminium chloride.

In a preferred embodiment A12 of the first aspect, the compound according to formula (I) is selected from the group consisting of

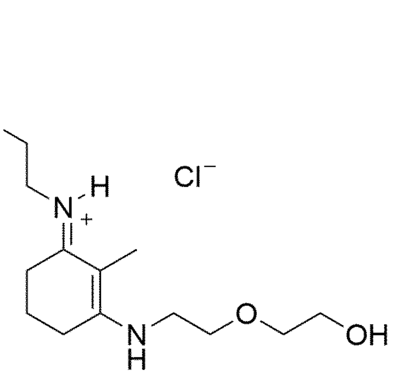

Formula (IIa)

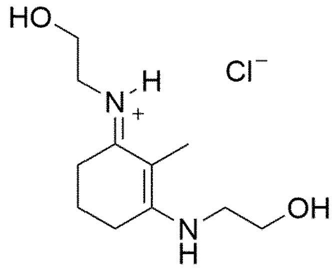

.

Formula (IIb)

In a second aspect, the present invention relates to a cosmetic composition, comprising a compound as defined in the first aspect (including all embodiments thereof as described herein) and optionally a dermatologically acceptable emulsifier, thickener, or emollient.

In a third aspect, the present invention relates to the compound as defined in the first aspect (including all embodiments thereof as described herein) or a cosmetic composition as defined in the second aspect for use in a sunscreen.

In a fourth aspect, the present invention relates to the use of a compound as defined in the first aspect (including all embodiments thereof as described herein) as a light stabilizer. In particular, the present invention is concerned with the use of a compound as defined in the first aspect (including all embodiments thereof as described herein) in household cleaning and treating agents as a light stabilizer.

In a fifth aspect, the present invention relates to the compound as defined in the first aspect (including all embodiments thereof as described herein) in body-care products for use as a light stabilizer.

Figure legend

Fig. 1 shows that all tested Compounds A to D absorb in relevant UV regions in view of skin protection. Further, it is depicted that Compound B possesses the highest specific extinction coefficient of all tested Compounds A to D.

Detailed Description

Before describing in detail exemplary embodiments of the present invention, definitions important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention

is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The term "compound(s) according to the invention", or "compounds of formula (I)" comprises the compound(s) as defined herein as well as a stereoisomer, salt, or tautomer thereof.

Depending on the substitution pattern, the compounds according to the invention may have one or more centers of chirality. The invention provides both the single pure enantiomers or pure diastereomers of the compounds according to the invention, and their mixtures and the use according to the invention of the pure enantiomers or pure diastereomers of the compounds according to the invention or their mixtures. Suitable compounds according to the invention also include all possible geometrical stereoisomers (cis/trans isomers or E/Z isomers) and mixtures thereof. Cis/trans isomers may e.g. be present with respect to an amide group. The term "stereoisomer(s)" encompasses both optical isomers, such as enantiomers or diastereomers, the latter existing due to more than one center of chirality in the molecule, as well as geometrical isomers (cis/trans isomers). The present invention relates to every possible stereoisomer of the compounds of formula (I), i.e. to single enantiomers or diastereomers, as well as to mixtures thereof.

The compounds of formula (I) may be amorphous or may exist in one or more different crystalline states (polymorphs) which may have different macroscopic properties such as stability or show different biological properties such as activities. The present invention relates to amorphous and crystalline compounds of formula (I), mixtures of different crystalline states of the respective compound of formula (I), as well as amorphous or crystalline salts thereof.

Salts of the compounds of the formula (I) may be pharmaceutically acceptable salts, such as those containing counterions present in drug products listed in the US FDA Orange Book database. They can be formed in a customary manner, e.g. by reacting the compound with an acid of the anion in question if the compound of formula (I) has a basic functionality.

Suitable anionic counterions are in particular chloride, bromide, hydrogensulfate, sulfate, dihydrogenphosphate, hydrogenphosphate, phosphate, nitrate, bicarbonate, carbonate, hexafluorosilicate, hexafluorophosphate, benzoate, and the anions of $C_1$-$C_4$-alkanoic acids, preferably formate, acetate, trifluoroacetate, propionate and butyrate, furthermore lactate, gluconate, and the anions of poly acids such as succinate, oxalate, maleate, fumarate, malate, tartrate and citrate, furthermore sulfonate anions such as besylate (benzenesulfonate), tosylate (p-toluenesulfonate), napsylate (naphthalene-2-sulfonate), mesylate (methanesulfonate), esylate (ethanesulfonate), and ethanedisulfonate. They can be formed by reacting compounds according to the invention that have a basic functionality with an acid of the corresponding anion. Preferred salts of the compounds of formula (I) are chloride salts.

Tautomers may be formed, if a substituent is present at the compound of formula (I), which allows for the formation of tautomers such as keto-enol tautomers or the like.

The organic moieties mentioned in the above definitions of the variables are - like the term halogen - collective terms for individual listings of the individual group members. The prefix $C_n$-$C_m$ indicates in each case the possible number of carbon atoms in the group.

The term "halogen" denotes in each case fluorine, bromine, chlorine, or iodine, in particular fluorine, chlorine, or bromine.

The term "halide" denotes in each case fluoride, bromide, chloride, or iodide, in particular fluoride, bromide, or chloride.

The term "alkyl" as used herein denotes in each case a straight-chain or branched alkyl group having usually from 1 to 5 carbon atoms, preferably from 1 to 4 carbon atoms. Examples of an alkyl group are methyl, ethyl, n-propyl, iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, 1,1-dimethylpropyl, and 1,2-dimethylpropyl. Methyl, ethyl, n-propyl, iso-propyl, and iso-butyl, are particularly preferred.

As used herein, the term "alkylene" refers to a linking straight-chain or branched alkylene group having usually from 1 to 4 carbon atoms, e.g. 1, 2, 3, or 4 carbon atoms. The alkylene group bridges a certain group to the remainder of the molecule. Preferred alkylene groups include methylene ($CH_2$), ethylene ($CH_2CH_2$), propylene ($CH_2CH_2CH_2$) and the like. A skilled person understands that, if it is referred, e.g., to $CH_2$ that the carbon atom being tetravalent has two valences left for forming a bridge ($-CH_2-$). Similarly, when it is referred, e.g., to $CH_2CH_2$, each carbon atom has one valence left for forming a bridge ($-CH_2CH_2-$). Furthermore, when it is referred, e.g., to $CH_2CH_2CH_2$, each terminal carbon atom has one valence left for forming a bridge ($-CH_2CH_2CH_2-$).

The term "alkoxy" as used herein denotes in each case a straight-chain or branched alkyl group which is bonded via an oxygen atom and has usually from 1 to 6 carbon atoms, preferably 1 to 2 carbon atoms, more preferably 1 carbon atom. Examples of an alkoxy group are methoxy, ethoxy, n-propoxy, iso-propoxy, n-butyloxy, 2-butyloxy, iso-butyloxy, tert.-butyloxy, and the like.

The term "($C_n$-$C_m$-alkyl)" as used herein denotes in each case a linker moiety, wherein the thereto attached

moieties are attached to the terminal carbons.

The term "C(=O)" as used therein denotes in each case a carbonyl moiety.

The term "C(=O)-($C_n$-$C_m$-alkyl)" as used herein denotes in each case an alkylcarbonyl, referring to a straight-chain or branched alkyl group as defined above, which is bonded via the carbon atom of a carbonyl group C(=O) to the remainder of the molecule.

The term "haloalkyl" as used herein denotes in each case a straight-chain or branched alkyl group having usually from 1 to 4 carbon atoms, preferably from 1 to 3 carbon atoms, especially 1 or 2 carbon atoms, wherein the hydrogen atoms of this group are partially or totally replaced with halogen atoms. Preferred haloalkyl moieties are selected from $C_1$-$C_4$-haloalkyl, more preferably from $C_1$-$C_3$-haloalkyl or $C_1$-$C_2$-haloalkyl, in particular from $C_1$-$C_2$-fluoroalkyl such as fluoromethyl, difluoromethyl, trifluoromethyl, 1-fluoroethyl, 2-fluoroethyl, 2,2-difluoroethyl, 2,2,2-trifluoroethyl, pentafluoroethyl, and the like. Trifluoromethyl is particularly preferred according to the invention.

The term "aryl" or "aromatic carbocycle" preferably includes 6-membered aromatic carbocyclic rings based on carbon atoms as ring members. A preferred example is phenyl. Unless otherwise indicated, the term "aryl" further covers "aromatic carbobicycles".

The term "aromatic carbobicycles" includes in general 6 to 14-membered, preferably 7- to 12-membered or 8- to 10-membered, more preferably 9- or 10-membered bicyclic rings comprising 6 to 14, preferably 7 to 12 or 8 to 10, more preferably 9 or 10 carbon atoms. In aromatic carbobicycles the Hückel (4n + 2) rule is fulfilled. Preferably, the term "aromatic" in connection with the carbobicyclic ring means that both rings of the bicyclic moiety are aromatic, so that, e.g., 8 $\pi$ electrons are present in case of a 10-membered aromatic carbobicyclic ring. A preferred example is naphthalene.

The term "saccharide" as used herein denotes in each case a carbohydrate having one or two sugar moieties, also referred to as monosaccharides or disaccharides. Monosaccharides are defined by the general formula $(C{\bullet}H_2O)_n$, wherein n is preferably between 3 to 7, in particular between 4 to 6. Glucose, galactose, fructose, and xylose may exemplarily be named. Disaccharides are defined in that two monosaccharides of the general formula $(C{\bullet}H_2O)_n$, wherein n is preferably between 3 to 7, in particular between 4 to 6, are connected via a glycosidic linkage. Sucrose, fructose, lactose, maltose, and trehalose may exemplarily be named.

The term "body-care product" refers to any product suitable to apply to the human body, e.g. sunscreen compositions, bath and shower products, preparations containing fragrances and odoriferous substances, hair-care products, dentifrices, decorative preparations, and cosmetic formulations containing active ingredients. Preferred are sunscreen compositions.

The term "sunscreen composition" or "sunscreen" or "skin-care product" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic UV filters, as well as other ingredients or additives, e.g., emulsifiers, emollients, viscosity regulators, stabilizers, preservatives, or fragrances.

The term "daily care composition" refers to any topical product, which reflects or absorbs certain parts of UV radiation and is used as an everyday care product for the human body, e.g., for face, body or hair. The daily care composition may comprise one or more active agents, e.g., organic or inorganic UV filters, as well as other ingredients or additives, e.g., emulsifiers, emollients, viscosity regulators, stabilizers, preservatives, or fragrances.

Suitable bath and shower additives are, e.g., shower gels, bath-salts, bubble baths and soaps. Suitable preparations containing fragrances and odoriferous substances are in particular scents, perfumes, toilet waters and shaving lotions (aftershave preparations).

Suitable hair-care products are, e.g., shampoos for humans and animals, in particular dogs, hair conditioners, products for styling and treating hair, perming agents, hair sprays and lacquers, hair gels, hair fixatives and hair dyeing or bleaching agents.

Suitable dentifrices are, e.g., tooth creams, toothpastes, mouth-washes, mouth rinses, antiplaque preparations and cleaning agents for dentures.

Suitable decorative preparations are, e.g., lipsticks, nail varnishes, eye shadows, mascaras, dry and moist make-up, rouge, powders, depilatory agents and suntan lotions.

Suitable cosmetic formulations containing active ingredients are, e.g., hormone preparations, vitamin preparations, vegetable extract preparations and antibacterial preparations.

The cited body-care products can be in the form of creams, ointments, pastes, foams, gels, lotions, powders, make-ups, sprays, sticks or aerosols. They preferably contain the compound of formula (I) in the aqueous phase. Thereby, the compound of formula (I) may acts as a light stabilizer.

The term "light stabilizers" as used herein are compounds suitable for protecting body-care and household cleaning and treating agents against photolytic degradation. The light stabilizer is usually present in the body-care product in a concentration of 50 to 1000 ppm.

The term "household cleaning and treating agent" refers to any product suitable to clean or treat household objects, e.g., liquid scouring agents, glass detergents, neutral cleaners (all-purpose cleaners), acid household cleaners (bath), WC cleaners, preferably in washing, rinsing and dishwashing agents, clear rinsing agents, dishwasher detergents, shoe polishes, polishing waxes, floor detergents and polishes, metal, glass and ceramic cleaners, textile-care products, agents for removing rust, color and stains (stain remover salt), furniture and multipurpose polishes and leather dressing agents (leather sprays).

Preferably, household cleaning agents are aqueous or alcoholic (e.g. ethanol or isopropyl alcohol) solutions of one or more of the following components: -anionic, nonionic, amphoteric and/or cationic surfactants -soaps, prepared by saponification of animal and vegetable greases -organic acids, like hydrochloric acid, phosphoric acid, or sulfuric acid, -for basic products inorganic (NaOH or KOH) or organic bases; -abrasives for improved cleaning of surfaces, - waxes and/or silicones for maintenance and protection of surfaces, -polyphosphates, - substances which eliminate hypochlorite or halogens; -peroxides comprising bleaching activators like TAED, for example sodium perborate or $H_2O_2$; -enzymes; -in washing detergents discoloration inhibitors, soil-release compounds, grey scale inhibitors, foam inhibitors, fluorescent whitening agents; -cleaning agents based on wax may comprise solvents selected from benzine, turpentine and/or paraffines and emulsifiers based on wax; -filling agents like silicates, polyphosphates, Zeolithes for powdery cleaning agents; -pigments, lakes or soluble dyes; -perfumes; and -light stabilizers, antioxidants and chelating agents.

The term "photostability" refers to the ability of a UV filter or any other molecule, which is exposed to sunlight, to stay stable upon irradiation. In particular, this means that the compound does not undergo a degradation process upon UV radiation.

The term "sun protection factor (SPF)" as used herein indicates how well the skin is protected by a sunscreen composition. In particular, the factor indicates how much longer the protected skin may be exposed to the sun without getting a sunburn in comparison to untreated skin. For example, if a sunscreen composition with an SPF of 15 is evenly applied to the skin of a person usually getting a sunburn after 10 minutes in the sun, the sunscreen allows the skilled person to stay in the sun 15 times longer. In other words, SPF 15 means that 1/15 of the burning UV radiation will reach the skin, assuming sunscreen is applied evenly at a thick dosage of 2 milligrams per square centimeter ($mg/cm^2$).

The term "critical wavelength" is defined as the wavelength at which the area under the UV protection curve (% protection versus wavelength) represents 90 % of the total area under the curve in the UV region (280-400 nm). For example, a critical wavelength of 370 nm indicates that the protection of the sunscreen composition is not limited to the wavelengths of UV-B, i.e. wavelengths from 280-320 nm, but extends to 370 nm in such a way that 90 % of the total area under the protective curve in the UV region are reached at 370 nm.

The term "ultraviolet filter" or "UV filter" as used herein refers to organic or inorganic compounds, which can absorb and/or reflect UV radiation caused by sunlight. UV filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UVA protection factor, which is at least one third of the labelled sun protection factor (SPF), e.g. if the sunscreen composition has an SPF of 30 the UVA protection factor has to be at least 10.

Preferred embodiments regarding the compound of formula (I) according to the present invention as well as the use of a compound of formula (I) in sunscreens or as light stabilizer are described hereinafter. It is to be understood that the preferred embodiments of the invention are preferred alone or in combination with each other. Further, preferred embodiments regarding the cosmetic composition comprising a compound of formula (I) according to the present invention as well as the use of such cosmetic composition in a sunscreen are described hereinafter.

As indicated above, the present invention relates in one embodiment to a compound of formula (I)

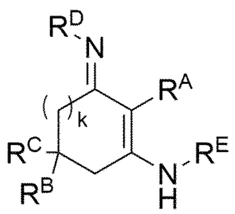

(I)

or a salt, stereoisomer, or tautomer thereof,
wherein

$R^A$ is H, $C_1$-$C_6$-alkyl, $C_1$-$C_6$-haloalkoxy, $C_2$-$C_6$-alkylene, (O-C(=O))-($C_1$-$C_4$-alkyl), (O-C(=O))-aryl, or S-($C_1$-$C_4$-alkyl);
$R^B$ is H, halogen, hydroxy, hydroxymethyl, $C_1$-$C_6$-alkyl, $C_2$-$C_6$-alkylene, or $C_1$-$C_6$-alkoxy;
$R^C$ is H, halogen, hydroxy, hydroxymethyl, $C_1$-$C_6$-alkyl, $C_2$-$C_6$-alkylene, or $C_1$-$C_6$-alkoxy;
$R^D$ is $(CH_2O)_n$-$R^M$, $(CH_2CH_2O)_n$-$R^M$, or $(CH_2CH_2CH_2O)_n$-$R^M$;
$R^E$ is $(CH_2O)_m$-$R^N$, $(CH_2CH_2O)_m$-$R^N$, or $(CH_2CH_2CH_2O)_m$-$R^N$;
$R^M$ is H, $C_1$-$C_4$-alkyl, or saccharide;
$R^N$ is H, $C_1$-$C_4$-alkyl, or saccharide;
n is an integer from 1 to 50;
m is an integer from 1 to 50; and
k is 0, 1, or 2.

Preferred embodiments regarding the compounds of formula (I), which are relevant for all aspects of the invention, are defined hereinafter.

In one embodiment of the present invention, the compound of formula (I) is in the form of the cosmetically acceptable salts thereof. In a preferred embodiment of the present invention, the compound of formula (I) is in the form of a salt according to formula (Ia)

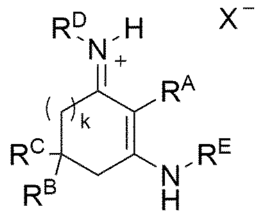

(Ia)

wherein
$X^-$ is halide, formate, acetate, succinate, oxalate, maleate, fumarate, malate, tartrate, citrate, trifluoroacetate, triflate, tosylate, or mesylate.

In a more preferred embodiment of the present invention, $X^-$ is fluoride, chloride, bromide, iodide, formate, acetate, oxalate, tartrate, tosylate, or mesylate. In an even more preferred embodiment of the present invention, $X^-$ is chloride, tosylate, or mesylate, in particular chloride.

In another embodiment of the present invention, k is 0 or 1, in particular 1.

In another embodiment of the present invention, $R^A$ is H, methyl, ethyl, propyl, iso-propyl, iso-butyl, tert-butyl, trifluoromethyl, (O-C(=O))-methyl, (O-C(=O)-phenyl, or thiomethyl. In a preferred embodiment of the present invention, $R^A$ is H, methyl, ethyl, propyl, iso-propyl, iso-butyl, or tert-butyl. In another preferred embodiment of the present invention, $R^A$ is methyl, ethyl, or thiomethyl, in particular methyl.

In one embodiment of the present invention, $R^B$ and $R^C$ are equal. In another embodiment of the present invention $R^B$ and $R^C$ are different. It is preferred that $R^B$ and $R^C$ are equal. In one embodiment of the present invention, $R^B$ is H, halogen, hydroxyl, hydroxymethyl, methyl, or methoxy and $R^C$ is H, halogen, hydroxyl, hydroxymethyl, methyl, or methoxy. In a preferred embodiment of the present invention, $R^B$ is H, halogen, hydroxyl, or hydroxymethyl, preferably H or halogen, and $R^C$ is H, halogen, hydroxyl, or hydroxymethyl, preferably H or halogen, in particular $R^B$ and $R^C$ are both H. In another preferred embodiment of the present invention, $R^B$ is H, halogen, $C_1$-$C_6$-alkyl, $C_2$-$C_6$-alkylene, or $C_1$-$C_6$-alkoxy, preferably H, halogen, $C_1$-$C_3$-alkyl, or $C_2$-$C_3$-alkylene, and $R^C$ is H, halogen, $C_1$-$C_6$-alkyl, $C_2$-$C_6$-alkylene, or $C_1$-$C_6$-alkoxy, preferably H, halogen, $C_1$-$C_3$-alkyl, or $C_2$-$C_3$-alkylene. In another preferred embodiment of the present invention, $R^B$ and $R^C$ are equal and are each selected from the group consisting of H, halogen, $C_1$-$C_6$-alkyl, $C_2$-$C_6$-alkylene, or $C_1$-$C_6$-alkoxy, preferably H, halogen, $C_1$-$C_3$-alkyl, or $C_2$-$C_3$-alkylene.

In one embodiment of the present invention, $R^D$ and $R^E$ are equal. In another embodiment of the present invention $R^D$ and $R^E$ are different.

In another embodiment of the present invention, $R^D$ is$(CH_2O)_n$-$R^M$ or $(CH_2CH_2O)_n$-$R^M$, wherein $R^M$ is H, methyl, ethyl, or iso-propyl, and wherein n is an integer from 1 to 30, preferable from 1 to 25, more preferably from 1 to 20, even more preferably from 1 to 15, in particular from 1 to10. In a preferred embodiment of the present invention, $R^D$ is $(CH_2CH_2O)_n$-$R^M$, wherein $R^M$ is H or methyl, and wherein n is an integer from 1 to 10.

In another embodiment of the present invention, $R^E$ is$(CH_2O)_m$-$R^N$ or $(CH_2CH_2O)_m$-$R^N$, wherein $R^N$ is H, methyl, ethyl, or iso-propyl, and wherein m is an integer from 1 to 30, preferable from 1 to 25, more preferably from 1 to 20, even more preferably from 1 to 15, in particular from 1 to10. In a preferred embodiment of the present invention, $R^E$ is $(CH_2CH_2O)_m$-$R^N$, wherein $R^N$ is H or methyl, and wherein m is an integer from 1 to 10.

In another embodiment of the present invention, $R^D$ is $(CH_2CH_2O)_n$-$R^M$, $R^E$ is $(CH_2CH_2O)_m$-$R^N$, wherein $R^M$

is H, $R^N$ is H, and wherein n is 1 or 2 and m is 1 or 2.

In another embodiment of the present invention, the compound according to formula (I) is selected from the group consisting of (E)-2-(2-hydroxyethoxy)-N-(3-((2-(2-hydroxyethoxy)ethyl)amino)-2-methylcyclohex-2-en-1-ylidene)ethan-1-aminium chloride and (E)-2-hydroxy-N-(3-((2-hydroxyethyl)amino)-2-methylcyclohex-2-en-1-ylidene)ethan-1-aminium chloride.

In another embodiment of the present invention, the compound according to formula (I) is selected from the group consisting of

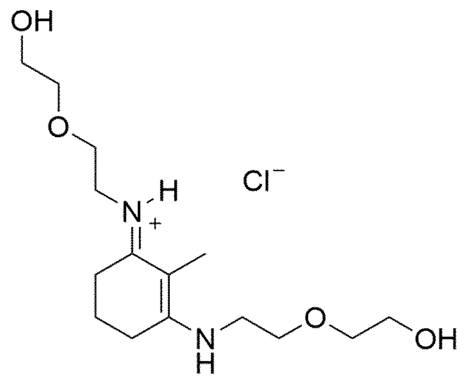

Formula (IIa)

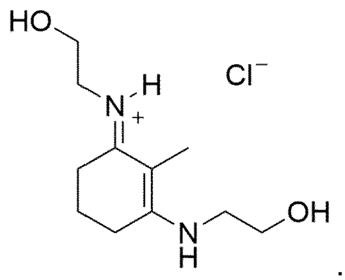

Formula (IIb)

In another aspect, the present invention relates in one embodiment to a cosmetic composition, comprising a compound as defined herein (including all embodiments thereof as described herein) and optionally a dermatologically acceptable emulsifier, thickener, or emollient.

In another embodiment of the present invention, the cosmetic composition further comprises an additional UV filter and/or light stabilizer, different to the compound of formula (I). The further UV filter and/or light stabilizer may be present in the water or the oil phase. In a preferred embodiment of the present invention, the additional UV filter is present in the oil phase. In connection with this embodiment, the cosmetic composition provides an even more improved protection against UV radiation since a UV filter is present in the oil phase and in the water phase, thus providing an improved repartition of the UV filters within the cosmetic composition.

In another embodiment of the present invention, the cosmetic composition further comprises at least two additional UV filter and/or light stabilizer, different to the compound of formula (I).

In another aspect, the present invention relates in one embodiment to the use of a compound as defined herein (including all embodiments thereof as described herein) or a cosmetic composition as defined herein (including all embodiments thereof as described herein) in a sunscreen. In connection with this embodiment, the compound as defined herein is used for protecting the skin from UV radiation.

In another aspect, the present invention relates in one embodiment to the use of a compound as defined in the first aspect (including all embodiments thereof as described herein) as a light stabilizer. In particular, the present invention is concerned with the use of a compound as defined in the first aspect (including all embodiments thereof as described herein) in body-care products and in household cleaning and treating agents as a light stabilizer. In connection with this embodiment, the compound as defined herein is used for protecting the body-care product or the household cleaning and treating agents from UV degeneration.

In another aspect, the present invention relates in one embodiment to the compound as defined in the first aspect (including all embodiments thereof as described herein) or a cosmetic composition as defined in the first aspect (including all embodiments thereof as described herein) herein for use in a sunscreen. In connection with this embodiment, the compound as defined herein is used for protecting the skin from UV radiation.

In another aspect, the present invention is relates in one embodiment to a compound as defined in the first aspect (including all embodiments thereof as described herein) in body-care products for use as a light stabilizer. In connection with this embodiment, the compound as defined herein is used for protecting the body-care product from UV degeneration.

In connection with the above embodiments, it is to be understood that the compounds of formula (I) are in particularly suitable for use as UV filter and hence for protection of the skin from UV radiation.

In connection with the above embodiments, it is to be understood that the compounds of formula (I) are further suitable for improving the photostability of body-care products and of household cleaning and treating agents. In particular, the compounds of formula (I) are suitable for improving the photostability of sunscreen compositions.

Furthermore, in connection with the above embodiments, it is to be understood that the sunscreen composition

may comprise at least one additive.

In one embodiment, the at least one additive is selected from the group consisting of emulsifier, emollients, viscosity regulators (thickeners), sensory enhancers, adjuvants, preservatives, and combinations thereof.

Preferred emulsifiers include

- glucose derivatives such as cetearyl glucoside, arachidyl glucoside, lauryl glucoside, polyglyceryl-3 methylglucose distearate, methyl glucose sesquistearate;
- sucrose derivative such as sucrose polystearate, sucrose palmitate;
- sorbitol derivatives;
- glycerides of fatty acids such as glyceryl stearate, glyceryl oleate;
- glumatic acid derivatives such as sodium stearoyl glutamate;
- sulfosuccinic acid derivatives such as disodium cetearyl sulfosuccinate;
- phosphoric acid derivatives such as potassium cetyl phosphate;
- fatty acid esters of polyglyceryl such as polyglyceryl-3-diisostearate, polyglyceryl-2-dipolyhydroxystearate;
- oxyalkenylated organomodified silicone / polysiloxane / polyalkyl / polyether copolymers and derivatives.

Preferred emollients include

- esters of linear or branched fatty acids with linear or branched fatty alcohols such as propylheptyl caprylate, coco caprylate, isopropyl myristate, ethylhexyl palmitate;
- esters of aromatic carboxylic acids with linear or branched fatty alcohols such as $C_{12}$-$C_{15}$-alkyl benzoate, ethylhexyl benzoate, phenethyl benzoate;
- dicarboxylic acid esters with linear or branched alcohols such as dibutyl adipate, dicaprylyl carbonate, diisopropyl sebacate;
- esters of hydroxycarboxylic acids with linear or branched fatty alcohols;
- esters of linear or branched fatty acids with polyhydric alcohol such as butylene glycol dicaprylate/dicaprate;
- mono-, di-, tri-glycerides based on $C_6$-$C_{18}$ fatty acids such as caprylic / capric triglycerides, coco glycerides;
- guerbet alcohols such as octyldodecynol;
- hydrocarbons such as hydrogenated polyisobutene, mineral oil, squalene, isohexadecane;
- ethers such as dicaprylyl ether;
- silicone derivatives (organomodified polysiloxanes) such as dimethylpolysiloxane, cyclic silicones.

Preferred thickeners include

- fatty alcohols such as cetyl alcohol, cetearyl alcohol, stearyl alcohol;
- fatty acids such as stearic acid;
- fatty acid esters such as myristyl stearate;
- waxes such as beeswax, carnauba wax, microcrystalline wax, ceresin, ozocerite;
- polysaccharides or derivatives such as xanthan gum, guar gum, agar gum, alginates, gellan gum, carraghenan;
- polyacrylates or homopolymers of reticulated acrylic acids or polyacrylamides such as carbomers, acrylate copolymers, acrylate / $C_{10}$-$C_{30}$-alkyl acrylate crosspolymer, acrylate / beheneth-25 methacrylate copolymer;
- silicate derivatives such as magnesium silicates;
- cellulose derivatives such as hydroxypropyl cellulose.

Preferred sensory enhancers include

- polyamide derivatives such as nylon-12;
- polymethyl methacrylates;
- silica;
- mica;
- polymethylsilsesquioxane;
- polyethylene;
- starch derivatives such as aluminum starch octenylsuccinate;
- dimethicone derivatives;
- boron nitride;
- HDI / trimethylol hexyllactone crosspolymer.

Preferred adjuvants include

- tocopherol derivatives;
- retinol derivatives;
- ascorbic acid derivatives;
- bisabolol;
- allantoin;
- panthenol;
- chelating agents (EDTA, EDDS, EGTA, phytic acid, piroctone olamine);
- ethylhexyl glycerin;
- caprylyl glycol;
- hydroxyacetophenone;
- caprylhydroxymic acid;
- propellants such as propane, butane, isobutene, dimethyl ether;
- styrene / PVP or styrene acrylamide copolymers;
- insect repellants such as butylacetylaminopropionate.

Preferred preservatives include

- phenoxyethanol;
- benzyl alcohol;
- methyl-, ethyl-, propyl-, butyl-, and isobutylparaben;
- zingerone.

Preferred perfumes are selected from the group consisting of limonene, citral, linalool, alpha-isomethylionon, geraniol, citronellol, 2-isobutyl-4-hydroxy-4-methyltetrahydropyrane, 2-tert.-pentylcyclohexylacetate, 3-methyl-5-phenyl-1-pentanol, 7-acetyl-1,1,3,4,4,6-hexamethyltetraline, adipine acid diester, alpha-amylcinnamaldehyde, alpha-methylionon, amyl C butylphenylmethylpropionalcinnamal, amylsalicylate, amylcinnamylalcohol, anisalcohol, benzoin, benzylalcohol, benzylbenzoate, benzylcinnamate, benzylsalicylate, bergamot oil, bitter orange oil, butylphenylmethylpropioal, cardamom oil, cedrol, cinnamal, cinnamylalcohol, citronnellylmethylcrotonate, lemon oil, coumarin, diethylsuccinate, ethyllinalool, eugenol, evernia furfuracea extracte, evernia prunastri extracte, farensol, guajak wood oil, hexylcinnamal, hexylsalicylate, hydroxycitronellal, lavender oil, lemon oil, linaylacetate, mandarine oil, menthyl PCA, methylheptenone, nutmeg oil, rosemary oil, sweet orange oil, terpineol, tonka bean oil, triethylcitrate, vanillin and combinations thereof.

In connection with the above preferred embodiments, it is to be understood that if the sunscreen or daily care composition comprises two or more additives, combinations of the additives as defined above are also part of the invention.

In connection with the above preferred embodiments, it is to be understood that the body-care product may further comprise water. In case water is present in the body-case product, the body-care product can be an oil in water emulsion (O/W emulsion) or a water in oil emulsion (W/O emulsion). According to a preferred embodiment of the present invention, the cosmetic composition comprising a compound of formula (I) is an O/W emulsion or a W/O emulsion.

The present invention is further illustrated by the following examples.

Examples

List of abbreviations

| | |
|---|---|
| °C | degrees Celsius |
| $\delta$ | chemical shift in parts per million downfield from tetramethylsilane |
| ES-MS | electrospray-mass spectrometry |
| 9 | gram(s) |
| Hz | hertz |
| *J* | coupling constant (in NMR spectrometry) |
| m | multiplet (spectral); meter(s); milli |
| Me | methyl |

(continued)

| | |
|---|---|
| MHz | megahertz |
| ml | milliliter |
| NMR | nuclear magnetic resonance |
| pH | potential of hydrogen; a measure of the acidity or basicity of an aqueous solution |
| rpm | revolutions per minute |
| rt | room temperature |
| T | temperature |
| TsO- | 4-methylbenzenesulfonate |

Example 1: Synthesis of compounds

Reagents and solvents were obtained from commercial sources and used without any further purification. Ion-exchange resin Amberlite IRA-402-Cl was used.

Chemical shifts are reported herein in parts per million (ppm) relative to residual solvent. Data for 1H-NMR are described as following: chemical shift (δ in ppm), multiplicity (s, singlet; d, doublet; t, triplet; q, quartet; m, multiplet), coupling constant (Hz), integration. Data for 13C-NMR are described in terms of chemical shift (δ in ppm).

The following analytic methods were used:
NMR spectra were acquired on a Bruker ARX-300 spectrometer (300 MHz and 75 MHz for 1H and 13C respectively) at a temperature of 298 K unless specified. ES-MS were obtained on an Ultra Performance Liquid Chromatography Acquity Waters with an interface to High Resolution Mass Spectrometry was performed in a Bruker Microtof-Q electrospray source in positive-ion mode.

Compound A having Formula (IIa): (E)-2-(2-hydroxyethoxy)-N-(3-((2-(2-hydroxyethoxy)ethyl)amino)-2-methylcyclohex-2-en-1-ylidene)ethan-1-aminium chloride; molecular formula: $C_{15}H_{29}ClN_2O_4$; molecular weight: 336.86

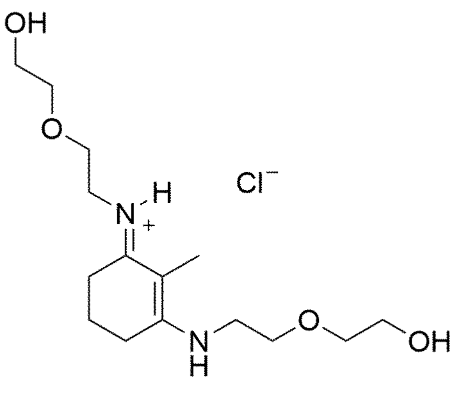

Formula (IIa)

To a stirring suspension of 2-methyl-1,3-cyclohexanedione (CAS: 1193-55-1) (1 equivalent) in dry toluene (200 ml), p-toluenesulfonic acid monohydrate (CAS: 6192-52-5) (1 equivalent) and 2-(2-aminoethoxy)ethanol (CAS: 929-06-6) (2.5 equivalents) were added and the mixture was heated to reflux for 48h with a Dean-Stark to remove the water produced during condensation. The mixture was cooled to rt and the excess of solvent removed under reduced pressure to yield a yellow oil. This oil was dissolved in water and 4-5 cycles with the ion-exchange resin Amberlite IRA-402-Cl were done, filtering the used resin in each cycle. After the ion-exchange is completed, water is removed under reduced pressure. None of the recrystallization conditions tried has been successful. Thus, Compound A is a brown oil with purity <90% (checked by NMR) and a total yield cannot be provided.

This procedure has been scaled up to 25g of the final compound, without any operational problems observed in the preparation.

$^1$H-NMR (300 MHz, deuterium oxide) δ 3.78 - 3.70 (m, 8H), 3.69 - 3.62 (m, 8H), 2.65 (t, *J* = 6.4 Hz, 4H), 1.91

(p, *J* = 6.5 Hz, 2H), 1.77 (s, 3H).

$^{13}$C-NMR (75 MHz, deuterium oxide) δ 170.15, 97.48, 71.81, 69.12, 60.35, 43.09, 25.10, 18.97, 7.85.

ES-MS (+) ($C_{15}H_{29}N_2O_4^+$): calc. 301.2122, found 301.2120.

**Compound B** having Formula (IIb): (E)-2-hydroxy-N-(3-((2-hydroxyethyl)amino)-2-methylcyclohex-2-en-1-ylidene)ethan-1-aminium chloride; molecular formula: $C_{11}H_{21}ClN_2O_2$; molecular weight: 248.75

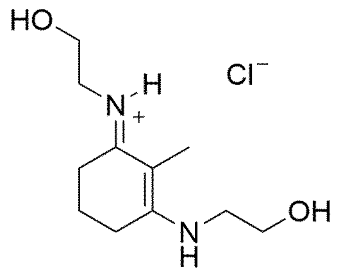

Formula (IIb)

To a stirring suspension of 2-methyl-1,3-cyclohexanedione (CAS: 1193-55-1) (1 equivalent) in dry toluene (200 ml), p-toluenesulfonic acid monohydrate (CAS: 6192-52-5) (1 equivalent) and 2-aminoethanol (CAS: 141-43-5) (2.5 equivalents) were added and the mixture was heated to reflux for 48h with a Dean-Stark to remove the water produced during condensation. The mixture was cooled to rt and the excess of solvent removed under reduced pressure to yield a yellow oil. The oil was dissolved in water and 4-5 cycles with the ion-exchange resin Amberlite IRA-402-Cl were done, filtering the used resin in each cycle. After the ion-exchange is completed, water is removed under reduced pressure and 2-3 washes with methanol (2ml) followed by acetone (100mi) were performed to yield a yellow-brown solid. Subsequent recrystallization from methanol by adding acetone yielded Compound B as a white-yellowish solid with a purity >95% (checked by NMR) and a total yield around 75%.

This procedure has been scaled up to 20g of the final compound, without any operational problems observed.

$^{1}$H-NMR (300 MHz, deuterium oxide) δ 3.76 (t, J= 5.4 Hz, 4H), 3.59 (t, J= 5.3 Hz, 4H), 2.65 (t, *J* = 6.3 Hz, 4H), 1.89 (q, *J* = 6.3 Hz, 2H), 1.78 (s, 3H).

$^{13}$C-NMR (75 MHz, deuterium oxide) δ 170.19, 97.40, 60.30, 45.32, 25.15, 19.03, 7.92. ES-MS (+) ($C_{11}H_{21}N_2O_2^+$): calc. 213.1598, found 213.1599.

**Comparative Compound C** ((E)-4-methoxy-N-(3-((4-methoxyphenyl)amino)-2-methylcyclopent-2-en-1-ylidene)benzenaminium 4-methylbenzenesulfonate) and D ((E)-2-bromo-N-(3-((2-bromophenyl)amino)-2-methylcyclopent-2-en-1-ylidene)benzenaminium 4-methylbenzenesulfonate) are accessible via the synthetic routes published by Losantos et al. in Angew. Chem. 2017, 129, 1-5 and are represented by the Formulae (IIIa) **(Compound C)** and (IIIb) **(Compound D).**

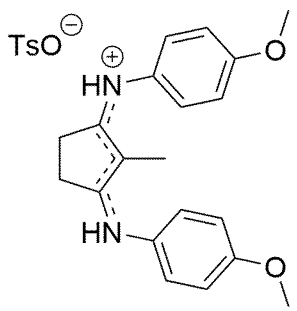

Formula (IIIa)

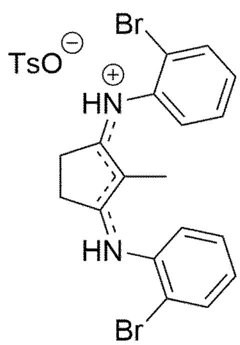

Formula (IIIb)

Example 2: Solubility test

The solubility of Compounds A to D was tested, wherein the addition of Compound A and B to the respective

solvent had been stopped after 50 % m/V. In case of Compound C and D, a precipitation after about 1 % m/V had been visible. The respective pH-values (see Table 1) had been measured of the final solution or suspension, respectively.

Table 1. Results of the solubility of Compounds A to D.

| Cosmetic solvent | Compound A | Compound B | Compound C | Compound D |
|---|---|---|---|---|
| Cetiol AB | insoluble | insoluble | < 1,2% | < 1,2% |
| Myritol 318 | insoluble | insoluble | < 1,0% | < 1,0% |
| Cetiol Ultimate | insoluble | insoluble | < 1,0% | < 1,0% |
| Water | > 50,0% (pH 6) | > 50,0% (pH 6) | < 1,0% (pH 7) | < 1,0% (pH 7) |

Example 3: Specific Extinction Coefficient

The specific extinction coefficient of Compounds A to D was measured using a Perkin Elmer Lambda 650 UV/VIS Spectrophotometer.

A stock solution of concentration c = 1mM of the UV-absorber was prepared in a 100ml volumetric flask using either buffer or ethanol. Dilution steps followed in order to adjust the concentration, such that the extinction E at optical pathlength d = 1 cm will be 1.0 ± 0.5. UV spectrum was determined in a cuvette of an optical pathlength of d = 1cm at 250nm to 500nm. With the Beer-Lambert law one obtains the molar decadic extinction coefficient at any wavelength with:

$$\varepsilon(\lambda) = \frac{E(\lambda)}{c \cdot d} \quad [\mathrm{l/(mol \cdot cm)}].$$

The respective specific extinction E%1,1cm can be obtained via the following equation:

$$E_{1,1}(\lambda) = \varepsilon(\lambda)[l/(mol \cdot cm)] \cdot \frac{10[g/l]}{M[g/mol]} \cdot 1[cm],$$

where M = molecular weight of the UV-absorber.

As can be seen in Figure 1, all tested compounds are absorbing in a UV region relevant for skin protection. Hence, all tested compounds may be applicable for a cosmetic sun care per se. The absorbance of Compound B is the highest.

A di-sodium hydrogen phosphate / potassium dihydrogen phosphate buffer (ordering number 1.09439.1000) or ethanol (ordering number 1.00983.2511), each purchased from Merck was used.

Table 2. Maximum absorbance of Compounds A to D.

| Tested Compounds | E (1%, 1 cm) |
|---|---|
| Compound A, buffer pH7 (phosphate buffer) | 943 |
| Compound B, buffer pH7 (phosphate buffer) | 1725 |
| Compound C, in EtOH | 737 |
| Compound D, in EtOH | 659 |

Example 4: Formulation of cosmetic compositions

Formulations 1 to 4 and a Placebo formulation were manufactured as follows. Part A and part B (see Table 3) were prepared separately and heated to 75-80 °C. Under increasing stirring, part B was incorporated to part A and homogenized with Ultra Turrax for 20 sec. at 11000rpm. The mixture was let cooled down to rt under stirring and part C was added. At rt pH 7-8 was adjusted with NaOH.

Table 3. Formulation of cosmetic compositions (Formulations 1 to 4 and Placebo formulation). INCI: International Nomenclature of Cosmetic Ingredients.

| Part | Trade name | *INCI* | Placebo | Formulation | | | |
|---|---|---|---|---|---|---|---|
| | | | | 1 | 2 | 3 | 4 |
| **A** | Amphisol K | *Potassium cetyl phosphate* | 1.80 | 1.80 | 1.80 | 1.80 | 1.80 |
| | Cutina GMS | *Glyceryl stearate* | 2.50 | 2.50 | 2.50 | 2.50 | 2.50 |
| | Lanette 18 | *Stearyl alcohol* | 2.50 | 2.50 | 2.50 | 2.50 | 2.50 |
| | Myritol 318 | *Caprylic/Capric Triglyceride* | 5.00 | 5.00 | 5.00 | 5.00 | 5.00 |
| | Cetiol AB | *C12-15 alkyl benzoate* | 10.00 | 10.00 | 10.00 | 10.00 | 10.00 |
| | Compound **C** | - | - | - | - | **2.00** | - |
| **B** | **Water** | ***Aqua*** | **70.10** | **68.10** | **70.10** | **68.10** | **65.10** |
| | 1,2 Propane diol | *1-2 propane diol* | 2.00 | 2.00 | 0.00 | 2.00 | 5.00 |
| | Rheocare XGN | *Xanthan Gum* | 0.30 | 0.30 | 0.30 | 0.30 | 0.30 |
| | Compound **A** | - | 0.00 | **2.00** | 0.00 | - | - |
| | Compound **B** | - | - | - | **2.00** | - | - |
| | Compound **D** | - | - | - | - | - | **2.00** |
| **C** | Cetiol Ultimate | *Undecane (and) Tridecane* | 5.00 | 5.00 | 5.00 | 5.00 | 5.00 |
| | Phenonip | *Phenoxyethanol (and) Methylparaben(and) Ethylparaben (and) Propylparaben (and) Butylparaben (and) Isobutylparaben* | 0.80 | 0.80 | 0.80 | 0.80 | 0.80 |
| | NaOH solution 30% | - | adjust pH to 7.0-8.0 (approx. 0.1g) | adjust pH to 7.0-8.0 (approx. 0.1g) | adjust pH to 7.0-8.0 (approx. 0.1g) | adjust pH to 7.0-8.0 (approx. 0.1g) | adjust pH to 7.0-8.0 (approx. 0.1g) |

Only in Formulations 1 and 2, the UV filters (Compounds A and B) could be incorporated into the water phase in sufficiently high concentrations. In contrast thereto, it had not been possible to solve the UV filter (Compound D) in the water phase of Formulation 4 in sufficiently high concentrations.

## Claims

**1.** A compound of formula (I)

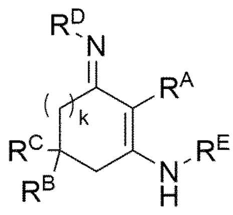

(I)

or a salt, stereoisomer, or tautomer thereof,
wherein

$R^A$ is H, $C_1$-$C_6$-alkyl, $C_1$-$C_6$-haloalkyl, $C_1$-$C_6$-haloalkoxy, $C_2$-$C_6$-alkylene, (O-C(=O))-($C_1$-$C_4$-alkyl), (O-C(=O))-aryl, or S-($C_1$-$C_4$-alkyl);
$R^B$ is H, halogen, hydroxy, hydroxymethyl, $C_1$-$C_6$-alkyl, $C_2$-$C_6$-alkylene, or $C_1$-$C_6$-alkoxy;
$R^C$ is H, halogen, hydroxy, hydroxymethyl, $C_1$-$C_6$-alkyl, $C_2$-$C_6$-alkylene, or $C_1$-$C_6$-alkoxy;
$R^D$ is $(CH_2O)_n$-$R^M$, $(CH_2CH_2O)_n$-$R^M$, or $(CH_2CH_2CH_2O)_n$-$R^M$;
$R^E$ is $(CH_2O)_m$-$R^N$, $(CH_2CH_2O)_m$-$R^N$, or $(CH_2CH_2CH_2O)_m$-$R^N$;
$R^M$ is H, $C_1$-$C_4$-alkyl, or saccharide;
$R^N$ is H, $C_1$-$C_4$-alkyl, or saccharide;
n is an integer from 1 to 50;
m is an integer from 1 to 50; and
k is 0, 1, or 2.

**2.** The compound according to claim 1, wherein it is in the form of a salt according to formula (Ia)

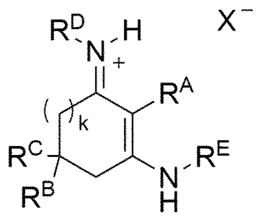

(Ia)

wherein
$X^-$ is halide, formate, acetate, succinate, oxalate, maleate, fumarate, malate, tartrate, citrate, trifluoroacetate, triflate, tosylate, or mesylate.

**3.** The compound according to claim 1 or 2, wherein

k is 1.

**4.** The compound according to any one of claims 1 to 3, wherein

$R^A$ is methyl, ethyl, or thiomethyl, preferably methyl.

**5.** The compound according to any one of claims 1 to 4, wherein

$R^D$ is $(CH_2CH_2O)_n$-$R^M$;
$R^M$ is H or methyl; and
n is an integer from 1 to 10.

**6.** The compound according to any one of claims 1 to 5, wherein

$R^E$ is $(CH_2CH_2O)_m$-$R^N$;
$R^N$ is H or methyl; and
m is an integer from 1 to 10.

**7.** The compound according to any one of claims 1 to 6, wherein

$R^D$ is $(CH_2CH_2O)_n$-$R^M$;
$R^E$ is $(CH_2CH_2O)_m$-$R^N$;
$R^M$ is H;
$R^N$ is H;
n is 1 or 2; and
m is 1 or 2.

**8.** The compound according to any one of claims 1 to 7, wherein $R^B$ and $R^C$ are equal, preferably H.

**9.** The compound according to any one of claims 2 to 8 wherein

$X^-$ is chloride, tosylate, or mesylate.

**10.** The compound according to claim 1, wherein the compound according to formula (I) is selected from the group consisting of (E)-2-(2-hydroxyethoxy)-N-(3-((2-(2-hydroxyethoxy)ethyl)amino)-2-methylcyclohex-2-en-1-ylidene)ethan-1-aminium chloride and (E)-2-hydroxy-N-(3-((2-hydroxyethyl)amino)-2-methylcyclohex-2-en-1-ylidene)ethan-1-aminium chloride.

**11.** The compound according to claim 1, wherein the compound according to formula (I) is selected from the group consisting of

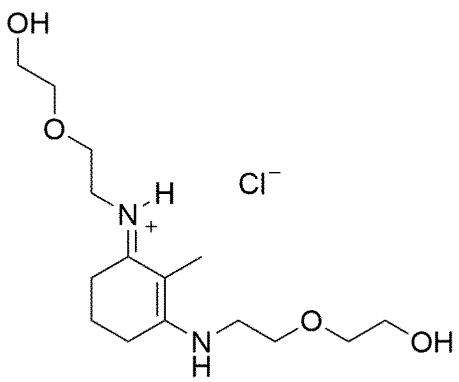

Formula (IIa)

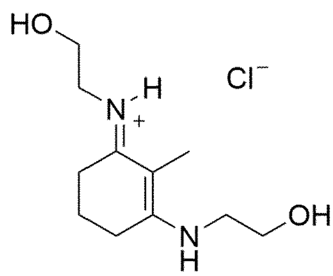

.

Formula (IIb)

**12.** A cosmetic composition, comprising a compound according to any one of claims 1 to 11 and optionally a dermatologically acceptable emulsifier, thickener, or emollient.

**13.** The compound according to any one of claims 1 to 11 or a cosmetic composition according to claim 12 for use in a sunscreen.

**14.** The compound according to any one of claims 1 to 11 in body-care products for use as a light stabilizer.

**15.** Use of a compound according to any one of claims 1 to 11 in household cleaning and treating agents as a light stabilizer.

**Patentansprüche**

**1.** Verbindung der Formel (I)

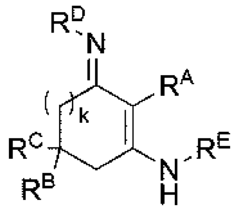

(I)

oder Salz, Stereoisomer oder Tautomer davon,
wobei
$R^A$ für H, $C_1$-$C_6$-Alkyl, $C_1$-$C_6$-Halogenalkyl, $C_1$-$C_6$-Halogenalkoxy, $C_2$-$C_6$-Alkylen, (O-C(=O))-($C_1$-$C_4$-Alkyl), (O-C(=O))-Aryl oder S-($C_2$-$C_4$-Alkyl) steht;
$R^B$ für H, Halogen, Hydroxy, Hydroxymethyl, $C_1$-$C_6$-Alkyl, $C_2$-$C_6$-Alkylen oder $C_1$-$C_6$-Alkoxy steht;
$R^C$ für H, Halogen, Hydroxy, Hydroxymethyl, $C_1$-$C_6$-Alkyl, $C_2$-$C_6$-Alkylen oder $C_1$-$C_6$-Alkoxy steht;
$R^D$ für $(CH_2O)_n$-$R^M$, $(CH_2CH_2O)_n$-$R^M$ oder $(CH_2CH_2CH_2O)_n$-$R^M$ steht;
$R^E$ für $(CH_2O)_m$-$R^N$, $(CH_2CH_2O)_m$-$R^N$ oder $(CH_2CH_2CH_2O)_m$-$R^N$ steht;
$R^M$ für H, $C_1$-$C_4$-Alkyl oder Saccharid steht;
$R^N$ für H, $C_1$-$C_4$-Alkyl oder Saccharid steht;
n für eine ganze Zahl von 1 bis 50 steht;
m für eine ganze Zahl von 1 bis 50 steht und
k für 0, 1 oder 2 steht.

**2.** Verbindung nach Anspruch 1, wobei sie in Form eines Salzes gemäß Formel (Ia)

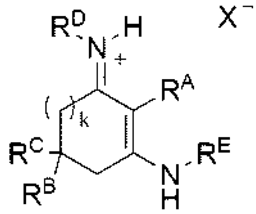

(Ia)

vorliegt, wobei
$X^-$ für Halogenid, Formiat, Acetat, Succinat, Oxalat, Maleat, Fumarat, Malat, Tartrat, Citrat, Trifluoracetat, Triflat, Tosylat oder Mesylat steht.

**3.** Verbindung nach Anspruch 1 oder 2, wobei k für 1 steht.

**4.** Verbindung nach einem der Ansprüche 1 bis 3, wobei $R^A$ für Methyl, Ethyl oder Thiomethyl, vorzugsweise Methyl, steht.

**5.** Verbindung nach einem der Ansprüche 1 bis 4, wobei

$R^D$ für $(CH_2CH_2O)_n$-$R^M$ steht;
$R^M$ für H oder Methyl steht und
n für eine ganze Zahl von 1 bis 10 steht.

**6.** Verbindung nach einem der Ansprüche 1 bis 5, wobei

$R^E$ für $(CH_2CH_2O)_m$-$R^N$ steht;
$R^N$ für H oder Methyl steht und
m für eine ganze Zahl von 1 bis 10 steht.

**7.** Verbindung nach einem der Ansprüche 1 bis 6, wobei

$R^D$ für $(CH_2CH_2O)_n$-$R^M$ steht;

$R^E$ für $(CH_2CH_2O)_m$-$R^N$ steht;
$R^M$ für H steht;
$R^N$ für H steht;
n für 1 oder 2 steht und
m für 1 oder 2 steht.

**8.** Verbindung nach einem der Ansprüche 1 bis 7, wobei $R^B$ und $R^C$ gleich sind und vorzugsweise für H stehen.

**9.** Verbindung nach einem der Ansprüche 2 bis 8, wobei $X^-$ für Chlorid, Tosylat oder Mesylat steht.

**10.** Verbindung nach Anspruch 1, wobei die Verbindung gemäß Formel (I) aus der Gruppe bestehend aus (E)-2-(2-Hydroxyethoxy)-N-(3-((2-(2-hydroxyethoxy)ethyl)amino)-2-methylcyclohex-2-en-1-yliden)ethan-1-aminiumchlorid und (E)-2-Hydroxy-N-(3-((2-hydroxyethyl)amino)-2-methylcyclohex-2-en-1-yliden)ethan-1-aminiumchlorid ausgewählt ist.

**11.** Verbindung nach Anspruch 1, wobei die Verbindung gemäß Formel (I) ausgewählt ist aus der Gruppe bestehend aus

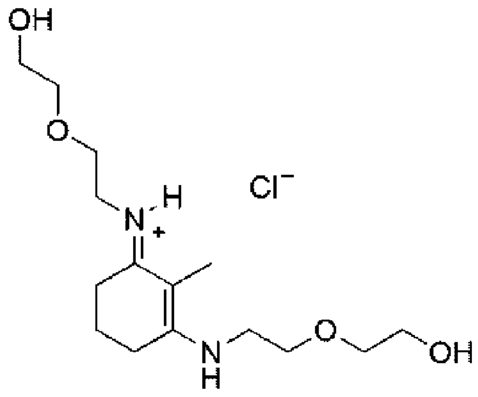

Formel (IIa)

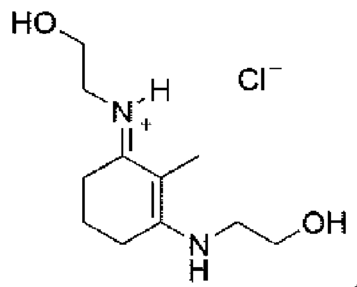

Formel (IIb)

**12.** Kosmetische Zusammensetzung, umfassend eine Verbindung nach einem der Ansprüche 1 bis 11 und gegebenenfalls einen dermatologisch unbedenklichen Emulgator, einen dermatologisch unbedenklichen Verdicker oder ein dermatologisch unbedenkliches Emolliens.

**13.** Verbindung nach einem der Ansprüche 1 bis 11 oder kosmetische Zusammensetzung nach Anspruch 12 zur Verwendung in einem Sonnenschutzmittel.

**14.** Verbindung nach einem der Ansprüche 1 bis 11 in Körperpflegeprodukten zur Verwendung als Lichtschutzmittel.

**15.** Verwendung einer Verbindung nach einem der Ansprüche 1 bis 11 in Haushaltsreinigungs- und -behandlungsmitteln als Lichtschutzmittel.

## Revendications

**1.** Composé de formule (I)

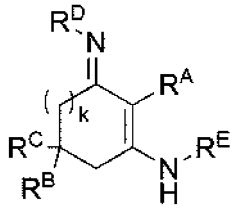

(I)

ou sel, stéréoisomère ou forme tautomère correspondant(e),
$R^A$ étant H, $C_1$-$C_6$-alkyle, $C_1$-$C_6$-halogénoalkyle, $C_1$-$C_6$-halogénoalcoxy, $C_2$-$C_6$-alkylène, (O-C(=O))-($C_1$-$C_4$-alkyle), (O-C(=O))-aryle, ou S-($C_1$-$C_4$-alkyle) ;
$R^B$ étant H, halogène, hydroxy, hydroxyméthyle, $C_1$-$C_6$-alkyle, $C_2$-$C_6$-alkylène, ou $C_1$-$C_6$-alcoxy ;
$R^C$ étant H, halogène, hydroxy, hydroxyméthyle, $C_1$-$C_6$-alkyle, $C_2$-$C_6$-alkylène, ou $C_1$-$C_6$-alcoxy ;
$R^D$ étant $(CH_2O)_n$-$R^M$, $(CH_2CH_2O)_n$-$R^M$, ou $(CH_2CH_2CH_2O)_n$-$R^M$ ;
$R^E$ étant $(CH_2O)_m$-$R^N$, $(CH_2CH_2O)_m$-$R^N$, ou $(CH_2CH_2CH_2O)_m$-$R^N$ ;
$R^M$ étant H, $C_1$-$C_4$-alkyle, ou saccharide ;
$R^N$ étant H, $C_1$-$C_4$-alkyle, ou saccharide ;
n étant un entier de 1 à 50 ;
m étant un entier de 1 à 50 ; et
k étant 0, 1, ou 2.

**2.** Composé selon la revendication 1, qui est sous la forme d'un sel selon la formule (Ia)

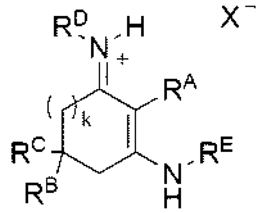

(Ia)

$X^-$ étant halogénure, formiate, acétate, succinate, oxalate, maléate, fumarate, malate, tartrate, citrate, trifluoroacétate, triflate, tosylate, ou mésylate.

**3.** Composé selon la revendication 1 ou 2,
k étant 1.

**4.** Composé selon l'une quelconque des revendications 1 à 3,
$R^A$ étant méthyle, éthyle, ou thiométhyle, préférablement méthyle.

**5.** Composé selon l'une quelconque des revendications 1 à 4,

$R^D$ étant $(CH_2CH_2O)_n$-$R^M$ ;
$R^M$ étant H ou méthyle ; et
n étant un entier de 1 à 10.

**6.** Composé selon l'une quelconque des revendications 1 à 5,

$R^E$ étant $(CH_2CH_2O)_m$-$R^N$ ;
$R^N$ étant H ou méthyle ; et
m étant un entier de 1 à 10.

**7.** Composé selon l'une quelconque des revendications 1 à 6,

$R^D$ étant $(CH_2CH_2O)_n$-$R^M$ ;
$R^E$ étant $(CH_2CH_2O)_m$-$R^N$ ;
$R^M$ étant H ;
$R^N$ étant H ;
n étant 1 ou 2 ; et
m étant 1 ou 2.

**8.** Composé selon l'une quelconque des revendications 1 à 7,
$R^B$ et $R^C$ étant égaux, préférablement H.

**9.** Composé selon l'une quelconque des revendications 2 à 8,

X⁻ étant chlorure, tosylate, ou mésylate.

**10.** Composé selon la revendication 1, le composé selon la formule (I) étant sélectionné dans le groupe constitué par chlorure de (E)-2-(2-hydroxyéthoxy)-N-(3-((2-(2-hydroxyéthoxy)éthyl)amino)-2-méthylcyclohex-2-én-1-ylidène)éthan-1-aminium et chlorure de (E)-2-hydroxy-N-(3-((2-hydroxyéthyl)amino)-2-méthylcyclohex-2-én-1-ylidène)éthan-1-aminium.

**11.** Composé selon la revendication 1, le composé selon la formule (I) étant sélectionné dans le groupe constitué par

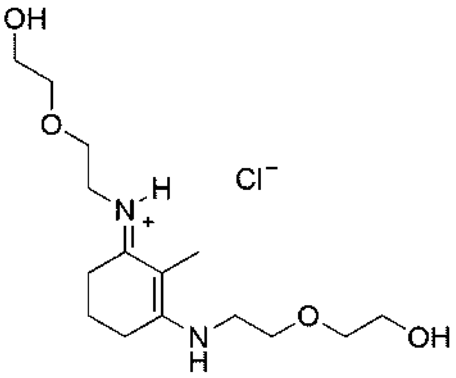

Formula (IIa)

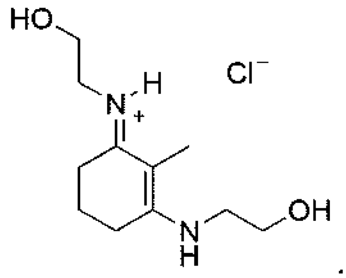

Formula (IIb)

**12.** Composition cosmétique, comprenant un composé selon l'une quelconque des revendications 1 à 11 et éventuellement un émulsifiant, un épaississant ou un émollient dermatologiquement acceptable.

**13.** Composé selon l'une quelconque des revendications 1 à 11 ou composition cosmétique selon la revendication 12 pour une utilisation dans un écran solaire.

**14.** Composé selon l'une quelconque des revendications 1 à 11 dans des produits de soin du corps pour une utilisation comme photostabilisant.

**15.** Utilisation d'un composé selon l'une quelconque des revendications 1 à 11 dans des agents de nettoyage ménager et de traitement comme photostabilisant.

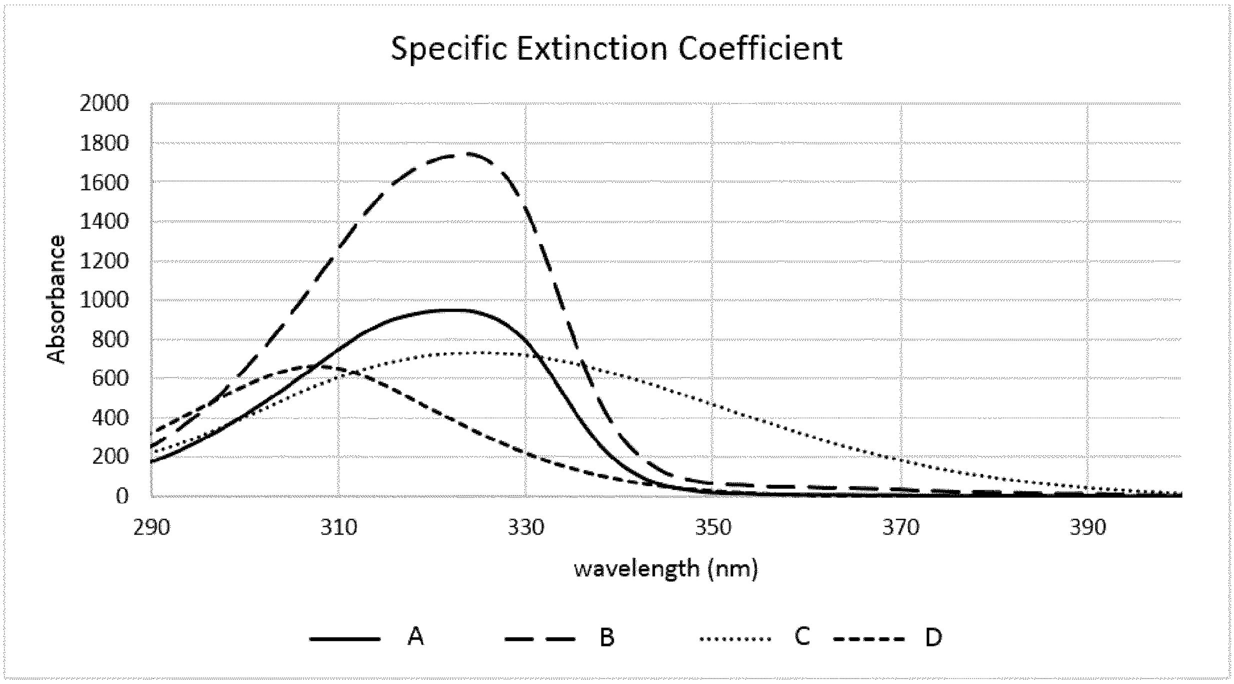
Specific Extinction Coefficient
Absorbance
2000
1800
1600
1400
1200
1000
800
600
400
200
0
290
310
330
350
370
390
wavelength (nm)
A
B
C
D

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- ES 2550374 B1


### Non-patent literature cited in the description


- **LOSANTOS et al.** *Angew. Chem.,* 2017, vol. 129, 1-5
- *CHEMICAL ABSTRACTS,* 1193-55-1
- *CHEMICAL ABSTRACTS,* 6192-52-5
- *CHEMICAL ABSTRACTS,* 929-06-6
- *CHEMICAL ABSTRACTS,* 141-43-5